# EUROPEAN PATENT SPECIFICATION

(11) **EP 1 892 007 B1**
(45) Date of publication and mention of the grant of the patent: **26.10.2011**
(21) Application number: 05751035.6
(22) Date of filing: 14.06.2005
(51) Int. Cl.: A61M 25/10

(54) **Balloon catheter**
Ballonkatheter
Cathéter à ballonnet

(43) Date of publication of application: 27.02.2008
(73) Proprietor: Vayu Co., Ltd, Nagoya-shi, Aichi 461-0023 (JP)
(72) Inventor: SHINKAI, Tomoyuki, c/o VAYU CO., LTD,, Higashi-ku Nagoya-shi (JP); SHIZU, Yasunori, Kasugai-shi, Aichi 486-0807 (JP); MIYATA, Akira, Shin-oe 1-chome,Kumamoto-shi, Kumamoto 862-097- (JP); TSUTSUI, Nobumasa, Nagoya-shi, Aichi 461-0023 (JP); TSUTSUI, Yasuhiro, Nagoya-shi, Aichi 461-0023 (JP)
(74) Representative: Intès, Didier Gérard André
(86) International application number: PCT/JP2005/010857
(87) International publication number: WO 2006/134638

(56) References cited:
- JP-A- 8 168 531
- JP-A- 63 238 875
- JP-A- 63 500 990

## Description

### TECHNICAL FIELD

The present invention relates to a balloon catheter used in percutaneous transluminal angioplasty. Particularly, the present invention relates to a balloon catheter used for expanding a curved blood vessel, an anastomotic site between an artificial blood vessel and an autologous blood vessel, a shunt anastomotic site created by a surgical reconstruction in a dialyzed patient, and a narrowed site in a blood vessel.

### BACKGROUND ART

Conventional balloon catheters used in percutaneous transluminal angioplasty include a type of balloon catheter having a straight-shaped balloon when inflated. In case of this type of balloon catheter having a straight-shaped balloon, the rigidity of the balloon when inflated is generally high, and the rigidity generally tends to be increased particularly when the balloon is inflated by higher pressure.

Therefore, if the balloon is excessively elongated, problems are likely to be caused: wherein, in an attempt to expand a curved blood vessel, the blood vessel is forcibly stretched: wherein both ends of the balloon are likely to locally impose strain on the wall of the outer portion of a curved blood vessel; and wherein the inner portion of the curved part of the balloon is bent and the balloon cannot be sufficiently inflated. In a case wherein the length of the balloon is shortened in order to lessen such problems, there has been a problem in that burden on a patient becomes large, since the balloon needs to be displaced several times and repeatedly inflated, if the range of a blood vessel to be expanded is longer than the length of the balloon.

Conventional balloon catheters also include another type of balloon catheter having a curved-shape balloon when inflated (for example, see Patent Documents 1, 2, and 3). In the techniques of such balloon catheters, a balloon is partially provided with a portion having a different stretchability, or a portion having a different film thickness. The portion having a small stretchability is disposed in the inner side of a curve and the portion having a large stretchability is disposed in the outer side. When the balloon is extended in the axial direction of the catheter, the balloon is curved.
Patent Document 1: Unexamined Japanese Patent Publication No. 10-286309
Patent Document 2: Description of U.S. Patent No. 6251093
Patent Document 3: Unexamined Japanese Patent Publication No. 2003-320031

Documents EP 0277 369, WO 87/01600and EP0 695 557 also deal with catheter, and particularly EP 0277369 and EP 0 695 557 deal with balloon catheter.

### DISCLOSURE OF THE INVENTION

### PROBLEMS TO BE SOLVED BY THE INVENTION

In the techniques disclosed in the above-described Patent Documents 1 and 2, the purpose to make a balloon curved is to change the direction or the position of the top end of a catheter. These techniques are different at least from a technique so as to perform vessel expansion in a curved blood vessel.

Moreover, although the technique disclosed in the above-described Patent Document 3 is meant for vessel expansion, there has been a problem as below described. The portion having a high stretchability and thin film thickness is inflated not only in the axial direction of the balloon, but also in the circumferential direction of the balloon. Optimizing the curved shape when the balloon is inflated is difficult, and, particularly, the optimizing of the curved shape needs to be performed by controlling the internal pressure of the balloon on a medical site. Therefore, handling of such balloon is difficult without a skilled operator.

Furthermore, in the technique disclosed in the above-described Patent Document 3, in consideration of providing with sufficient resistance to pressure to the portion having a high stretchability and the portion having a thin film thickness, the film thickness cannot be excessively small. Therefore, there has been a problem in that selecting a material, having a sufficiently high strechability and, in addition, a sufficient resistance to pressure, is difficult.

The present invention is made so as to solve the above-described problems. The purpose of the present invention is to provide a balloon catheter capable of expanding a narrowed site of a blood vessel in the vicinity of a curved part of the blood vessel, and of adapting the shape of the balloon to the shape of the curved part of the blood vessel without performing delicate control.

### MEANS FOR SOLVING THE PROBLEMS

The structure of the present invention is defined in claim 1.
A balloon catheter according to the present invention includes a long shaft having an inner pipe and an outer pipe, and a balloon provided at a distal end of the shaft. A distal end of the balloon is connected to the inner pipe in a fluid-tight manner. A proximal end of the balloon is connected to the outer pipe in the fluid-tight manner. The balloon is inflated and deflated in accordance with pressure of fluid supplied to an inside of the balloon through a space formed between the inner pipe and the outer pipe. The balloon is molded in advance such that the balloon is inflated by pressure only to an extent in which a film of the balloon is not stretched, such that the balloon becomes a curved shape having a curved part between the proximal end and the distal end of the balloon, and such that the balloon, when deflated, is folded so as to be in a straight shape, in which the curved part is hidden, and wound around the inner pipe.

According to the balloon catheter constituted as above, the shape of the balloon when inflated has a curved part between the proximal end and the distal end of the balloon, which allows the balloon to be inflated at a proper indwelling position even in a largely curved blood vessel without forcibly bending the balloon. Therefore, even if a blood vessel is largely curved, appropriate angioplasty can be performed on a narrowed site. In addition, unlike the case of a straight-shaped balloon, the top end of the balloon is adapted so as not to be pressed against a blood vessel wall. Therefore, damage to a blood vessel wall is not likely to be caused, and a curved blood vessel is not likely to be forcibly stretched and straighten.

Moreover, although the shape of the balloon becomes a curved shape when the balloon is inflated, the balloon when deflated is folded so as to be in a straight shape, in which the curved part is hidden, and wound around the inner pipe. Therefore, the shape of the balloon when deflated does not disturb insertion of the balloon into a blood vessel.

Furthermore, the balloon is molded in advance so as to have a desired curved shape, and inflated by pressure only to an extent in which a film of the balloon is not stretched. Unlike the case of a balloon in which the angle of the curved part is changed in accordance with pressure, the curved shape, formed when the balloon is inflated, can be easily optimized. Therefore, effort to delicately control the inner pressure of the balloon is not necessary on a medical practice site, which allows the balloon to be handled without a skilled operator. Moreover, the balloon does not have to be provided with a portion having a high stretchability, or a portion having a small film thickness. Therefore, the entire balloon can be made of the same material and with a uniform film thickness, and the material for producing the balloon can be easily selected.

In the balloon catheter constituted as above, the balloon is preferably made of a material having a stretchability no greater than 200% when the balloon is inflated by pressure of 1.5MPa. By making the balloon with such material, excessive expansion of the balloon when inflated can be inhibited. If the balloon if formed with a material having a stretchability of over 200% and when the balloon is inflated, the film of the balloon is likely to be excessively expanded. In this case, if pressurization is simply performed, the shape of the balloon is likely to be different from the desired shape. Therefore, there is a possibility that delicate control of the internal pressure becomes necessary.

An example of such material of the balloon is selected from a group of polyamide, polyamide elastomer, polyethylene terephthalate, polyester elastomer, and polyurethane. Particularly, the balloon is preferably made of a material having properties in which a tensile strength is 30N/cm² and above, a stretchability is no greater than 600%, and Shore hardness D is 50 and above.

The curved part of the balloon is formed in advance so as to have a specific angle. The degree of the specific angle may be arbitrarily determined. Generally, some types of balloons having different angles are prepared so that the balloon having an optimal angle that matches the angle of the blood vessel of a patient can be selected. Examples of the angles of the curved parts of such balloons which should be prepared may be determined to be between 45-135°.

### BRIEF DESCRIPTION OF THE DRAWING

[Fig. 1] is a side view of a balloon catheter according to a preferred embodiment of the invention; and
[Figs. 2(a)-(d)] are explanatory views illustrating the states of a balloon from a deflated state to an inflated state, in which Fig. 2(a) shows the deflated state, Fig. 2(b) shows a transient state from the deflated state to the inflated state, Fig. 2(c) shows the inflated state, and Fig. 2(d) shows a state wherein pressure is further applied in the inflated state.

### EXPLANATION OF REFERENTIAL NUMERAL

1... balloon catheter, 3...shaft, 5... balloon, 7...connecter, 11...oxter pipe, 13...inner pipe, 15...pressure fluid supply port, 17...guidewire insertion port, 21...first lumen, 22...second lumen, 25...marker

### DETAILED DESCRIPTION OF PREFERRED EMBODIMENTS OF THE INVENTION

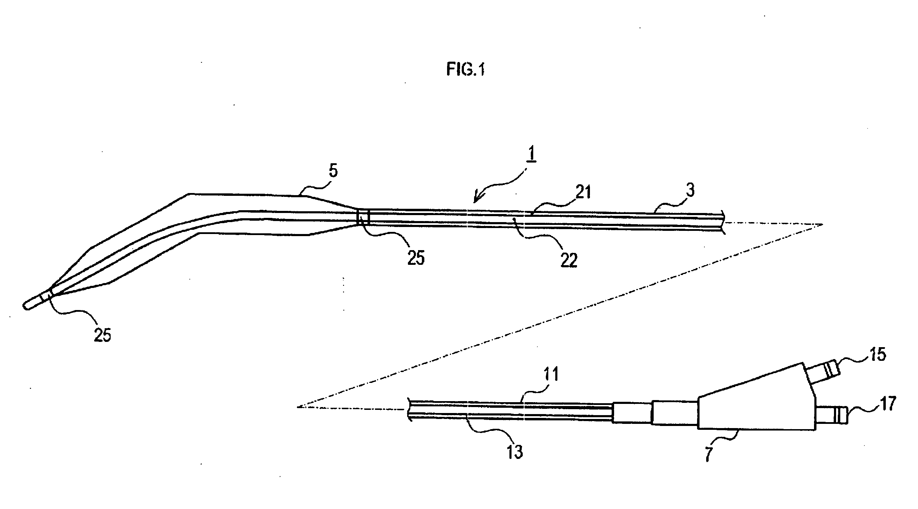

The following describes an embodiment of the present invention by way of an example.
A balloon catheter 1 shown in Fig. 1 includes a long shaft 3, a balloon 5 provided at the distal end of the shaft 3, and a connector 7 provided at the proximal end of the shaft 3.

The shaft 3 is a double pipe constituted by inserting an inner pipe 13 into the internal hollow of an outer pipe 11. The distal end of the outer pipe 11 is connected to the proximal end of the balloon 5 in a fluid-tight manner, and the internal hollow of the outer pipe 11 communicates with the inside of the balloon 5. The inner pipe 13 extends toward the distal direction further than the connection between the outer pipe 11 and the balloon 5, and passes through the inside of the balloon 5. The distal end of the balloon 5 is connected to the outer peripheral surface of the distal end of the inner pipe 13 in a fluid-tight manner.

The outer pipe 11 is a tube material, made of polyamide (polyamide 12), and having an outer diameter determined to be 1.3mm. Materials for forming the outer pipe 11, other than polyamide, may be polyethylene, polyimide, polyetheretherketone, polyethylene terephthalate, polyurethane or polypropylene.

The inner pipe 13 is a tube material made of high-density polyethylene. Materials for forming the inner pipe 13, other than high-density polyethylene, may be polyamide, polyimide, polyetheretherketon, polyethylene terephthalate, polyurethane, polypropylene or fluoroplastic.

The balloon 5 is a hollow body formed by a film made of polyamide (polyamide 12), and is adopted so as to be inflated and deflated in accordance with the pressure of fluid supplied to the inside of the balloon 5. As shown in Fig. 1, when the balloon 5 is inflated, the balloon 5 has a shape having a curved part between the proximal end and the distal end of the balloon 5. The curve direction and the angle of the curved part are determined by the shape of the process mold (die) used when the balloon 5 is formed. The angle is determined to be a specific angle selected from in range of 45-135°. If a balloon is made of a film material that can be easily stretched, the balloon film is stretched in accordance with pressurization, and, as a result, the curve direction and the angle of the above-described curved part are likely to be changed. Therefore, in the present embodiment, the balloon film is made of a material, having a stretchability no greater than 200% when the balloon is inflated by pressure of 1.5MPa, so as to limit the stretching of the balloon film, and to maintain the curve direction and the angle of the curved part. An example of such material is any of polyamide, polyamide elastomer, polyethylene terephthalate, polyester elastomer, and polyurethane. Particularly, a preferable material has properties in which a tensile strength is 30N/cm² and above, a stretchability is no greater than 600%, and Shore hardness is 50D and above. The balloon 5 has an outer diameter selected from one of 3.0mm, 4.0mm, and 5.0mm, and a length selected from one of 20mm and 40mm. Balloons 5 in different sizes are prepared so that one of the balloons 5 in the optimal size can be selected in accordance with the vascular diameter and the length of a narrowed site of a patient. Materials used for forming the balloon 5 a may be polyamide, polyamide elastomer, polyethylene terephthalate, polyester elastomer, and polyurethane.

The connector 7, which is a member used for connecting the balloon catheter 1 and a supply source of pressure fluid (not shown), is provided with a pressure fluid supply port 15 and a guidewire insertion port 17. Respective proximal ends of the outer pipe 11 and the inner pipe 13 are connected to the connector 7. The space (hereinafter also referred to as the "first lumen 21") between the inner periphery of the outer pipe 11 and the outer periphery of the inner pipe 13 communicates with the pressure fluid supply port 15 of the connector 7, and the internal hollow (hereinafter also referred to as the "second lumen 22") of the inner pipe 13 communicates with the guidewire insertion port 17 of the connector 7.

In the vicinity of both ends of the balloon 5, metal markers 25 are mounted around the outer periphery of the inner pipe 13.
In the balloon catheter 1 constituted as above, when the pressure fluid is supplied through the pressure fluid supply port 15 of the connector 7, the pressure fluid is introduced to the inside of the balloon 5 through the internal hollow of the shaft 3 (the first lumen 21) and the balloon 5 is inflated, while when the pressure fluid inside the balloon 5 is drained, the balloon 5 is deflated.

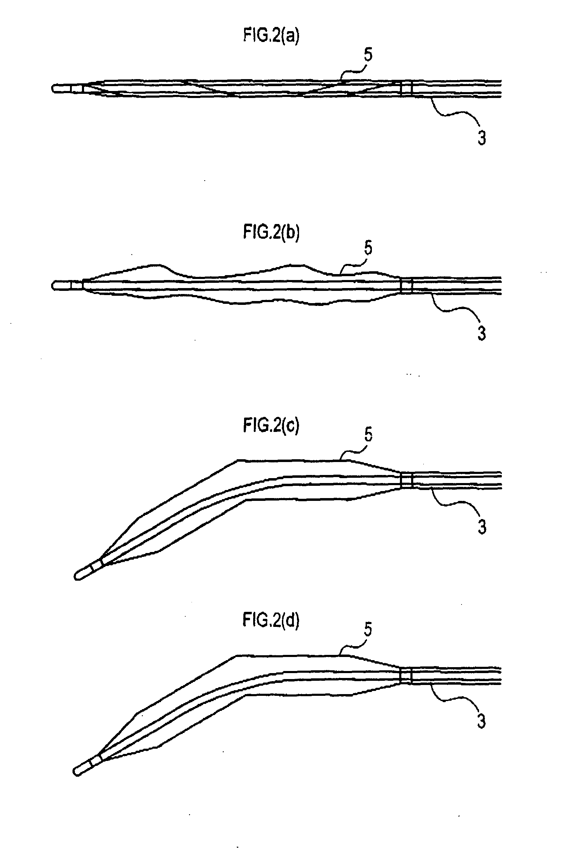

When the balloon catheter 1 is inserted into a blood vessel, the balloon 5 is in a deflated state. In this state, the balloon 5, which is formed in advance in a curved shape, is folded and wound around the inner pipe 13 having a rigidity higher than the rigidity of the balloon 5 so that a linear shape is maintained (see Fig. 2(a)). Subsequently, while a guidewire, inserted and placed in the blood vessel beforehand, is introduced into the inner pipe 13, the balloon catheter 1 is inserted along the guidewire to a target blood vessel. When it is checked that the balloon 5 is inserted up to a narrowed site in a curved blood vessel, the balloon 5 is inflated by the pressure of liquid.

When liquid is supplied to the inside of the balloon 5 so as to inflate the balloon 5 by pressure, the balloon 5, folded and wound around the inner pipe 13, is gradually unfolded as shown in Fig. 2(b). When the liquid is supplied as much as the capacity of the balloon 5, the balloon 5 becomes a curved shape (see Fig. 2(c)) which is the shape when the balloon 5 is molded.

Subsequently, if the supplying pressure of the liquid is higher, the internal pressure of the balloon 5 is increased and the tension of the balloon film becomes higher. However, as described above, the balloon film is made of a material, having a stretchability no greater than 200% when the balloon 5 is inflated by pressure of 1.5MPa. Therefore, even if the internal pressure of the balloon 5 is increased, the curved shape of the balloon 5 is maintained in a shape wherein the angle of the curved part is not increased and the diameter of the balloon 5 is not enlarged (see Fig. 2(d)).

According to the balloon catheter 1 as described above, the shape of the balloon 5 when inflated has a curved part between the proximal end and the distal end of the balloon 5, which allows the balloon 5 to indwell at a proper indewelling position even in a largely curved blood vessel without forcibly bending the balloon 5. Therefore, appropriate angioplasty can be performed on a narrowed site of a curved blood vessel. Unlike a straight-shaped balloon, since the balloon 5 is designed such that the top end of the balloon 5 is not pressed against a blood vessel wall during angioplasty, the balloon 5 less likely damages a blood vessel wall, or undesirably stretches the curving of a blood vessel.

Moreover, even if the internal pressure of the balloon 5 is increased, the curve angle of the balloon 5 is maintained, and the length and the diameter of the balloon 5 are not substantially changed. Therefore, unlike a balloon whose curve angle and the diameter are changed in accordance with the internal pressure, the balloon 5 can be inflated so as to become a predetermined curved shape without delicately controlling the internal pressure of the balloon 5. As a result, the curved shape of the inflated balloon 5 can be easily optimized, extra effort is not required for delicately controlling the inner pressure of the balloon 5 on a medical site, which allows the balloon 5 to be easily handled without a skilled operator.

In addition, since the balloon does not need to be provided with a portion having a high stretchability or a smaller film thickness, the entire balloon 5 can be made of the same material and with a uniform film thickness. Therefore, a material for producing the balloon 5 can be easily selected.

Although the present invention has been described with respect to a preferred embodiment, the present invention should not be limited to the above-described embodiment and can be embodied in various forms.
For example, although the balloon 5, having a specific curve angle, is shown in the above-described embodiment, the angle of the balloon 5 may be arbitrarily determined. Moreover, the size of the shaft 3, the size of the balloon 5, and so on may also be arbitrarily determined.

## Claims

1. A balloon catheter comprising:
a long shaft (3) including an inner pipe (13) and an outer pipe (11); and
a balloon (5) provided at a distal end of the shaft (3), a distal end of the balloon (5) being connected to the inner pipe (13) in a fluid-tight manner, a proximal end of the balloon (5) being connected to the outer pipe (11) in the fluid-tight manner, and the balloon (5) being inflated and deflated in accordance with pressure of fluid supplied to an inside of the balloon (5) through a space formed between the inner pipe (13) and the outer pipe (11),
**characterized in that** the balloon (5) is molded in advance such that the balloon (5) becomes a curved shape having a curved part between the proximal end and the distal end of the balloon (5) when inflated by pressure only to an extent in which a film of the balloon (5) is not stretched, and the balloon (5) is folded and wound around the inner pipe (13), when deflated, so as to be in a straight shape, in which the curved part is hidden,
and **in that** the inner pipe (13) presents a straight shape when the balloon (5) is deflated, and a curved shape when the balloon (5) is inflated.

2. The balloon catheter according to claim 1 wherein the balloon (5) is made of a material having a stretchability no greater than 200% when the balloon (5) is inflated by pressure of 1.5MPa.

3. The balloon catheter according to claim 1 or 2 wherein the balloon (5) is made of a material selected from a group of polyamide, polyamide elastomer, polyethylene terephthalate, polyester elastomer, and polyurethane.

## Patentansprüche

1. Ballonkatheter umfassend:
einen langen Schaft (3) mit einem inneren Rohr (13) und einem äußeren Rohr (11) und
einen Ballon (5), der an einem distalen Ende des Schafts (3) vorgesehen ist, wobei ein distales Ende des Ballons (5) auf fluiddichte Weise mit dem inneren Rohr (13) verbunden ist, ein proximales Ende des Ballons (5) auf fluiddichte Weise mit dem äußeren Rohr (11) verbunden ist und der Ballon (5) aufgebläht und entleert wird gemäß dem Druck des einem Inneren des Ballons (5) durch einen zwischen dem inneren Rohr (13) und dem äußeren Rohr (11) gebildeten Raum zugeführten Fluids,
**dadurch gekennzeichnet,**
**daß** der Ballon (5) im voraus so geformt ist, daß der Ballon (5) eine gekrümmte Form mit einem gekrümmten Abschnitt zwischen dem proximalen Ende und dem distalen Ende des Ballons (5) annimmt, wenn er durch Druck nur bis zu einem Ausmaß aufgebläht wird, in welchem eine Folie des Ballons (5) nicht gereckt wird, und der Ballon (5) im entleerten Zustand gefaltet und um das innere Rohr (13) gewunden ist, um in einer geraden Form zu sein, in welcher der gekrümmte Abschnitt verborgen ist, und
**daß** das innere Rohr (13), wenn der Ballon (5) im entleerten Zustand ist, eine gerade Form und, wenn der Ballon (5) im aufgeblähten Zustand ist, eine gekrümmte Form besitzt.

2. Ballonkatheter nach Anspruch 1, wobei der Ballon (5) aus einem Material besteht, das eine Dehnbarkeit von nicht mehr als 200 % aufweist, wenn der Ballon (5) mit einem Druck von 1,5 MPa aufgebläht wird.

3. Ballonkatheter nach Anspruch 1 oder 2, wobei der Ballon (5) aus einem Material hergestellt ist, das aus einer aus Polyamid, Polyamidelastomer, Polyethylenterephthalat, Polyesterelastomer und Polyurethan bestehenden Gruppe ausgewählt ist.

## Revendications

1. Cathéter à ballonnet comportant :
une tige longue (3) comprenant un tuyau intérieur (13) et un tuyau extérieur (11) ; et
un ballonnet (5) prévu à une extrémité distale de la tige (3), une extrémité distale du ballonnet (5) étant reliée au tuyau intérieur (13) d'une manière étanche aux fluides, une extrémité proximale du ballonnet (5) étant reliée au tuyau extérieur (11) de manière étanche aux fluides, et le ballonnet (5) étant gonflé et dégonflé en fonction d'une pression d'un fluide délivrée à un intérieur du ballonnet (5) à travers un espace formé entre le tuyau intérieur (13) et le tuyau extérieur (11),
**caractérisé en ce que** le ballonnet (5) est moulé à l'avance de telle sorte que le ballonnet (5) devient d'une forme courbe ayant une partie courbe entre l'extrémité proximale et l'extrémité distale du ballonnet (5) lorsqu'il est gonflé par une pression seulement dans une mesure telle qu'un film du ballonnet (5) n'est pas étiré, et le ballonnet (5) est plié et enroulé autour du tuyau intérieur (13), lorsqu'il est dégonflé, de façon à être d'une forme droite, dans laquelle la partie courbe est dissimulée,
et **en ce que** le tuyau intérieur (13) présente une forme droite quand le ballonnet (5) est dégonflé, et une forme courbe quand le ballonnet (5) est gonflé.

2. Cathéter à ballonnet selon la revendication 1 dans lequel le ballonnet (5) est fabriqué dans un matériau ayant une capacité d'extension qui n'est pas plus supérieure à 200 % quand le ballonnet (5) est gonflé à une pression de 1,5 MPa.

3. Cathéter à ballonnet selon la revendication 1 ou 2 dans lequel le ballonnet (5) est fabriqué dans un matériau choisi parmi un groupe comprenant un polyamide, un élastomère de polyamide, un téréphtalate de polyéthylène, un élastomère de polyester, et un polyuréthane.
